# EUROPEAN PATENT APPLICATION

(11) **EP 3 293 261 A1**
(43) Date of publication of application: **14.03.2018**
(21) Application number: 17188275.6
(22) Date of filing: 29.08.2017
(51) Int. Cl.: C12N 9/88, C12P 19/04, C12N 15/63

(54) **GLUCONACETOBACTER HAVING CELLULOSE PRODUCTIVITY**

(30) Priority: 12.09.2016 KR 20160117368
(71) Applicant: Samsung Electronics Co., Ltd., Gyeonggi-do 16677 (KR)
(72) Inventor: CHUNG, Soonchun, Suwon-si Gyeonggi-do 16678 (KR); YUN, Jiae, Suwon-si Gyeonggi-do 16678 (KR); KANG, Jinkyu, Suwon-si Gyeonggi-do 16678 (KR); PARK, Jinhwan, Suwon-si Gyeonggi-do 16678 (KR)
(74) Representative: Grünecker Patent- und Rechtsanwälte PartG mbB

(57) **Abstract**

Provided is a microorganism of the genus *Gluconacetobacterwhich* has cellulose productivity due to overexpression of fructose-bisphosphate aldolase and optionally, phosphoglucomutase, UTP-glucose-1-phosphate uridylyltransferase, or cellulose synthase. Further, a method of producing cellulose and a method of producing the microorganism are provided.

<110>    Samsung Electronics Co., Ltd.

<120>    Gluconacetobacter Having Enhanced Cellulose Productivity

<130>    EP113088FZSEpau

<140>    not yet assigned
<141>    herewith

<150>    KR10-2016-0117368
<151>    2016-09-12

<160>    19

<170>    KopatentIn 2.0

<210>    1
<211>    362
<212>    PRT
<213>    Gluconacetobacter xylinus DSM2325 M9

<400>    1
Met Ala His Ser Ala Arg Pro Ser Leu Pro Pro Gly Val Val Thr Gly
  1               5                  10                  15

Glu Asn Tyr Arg Lys Leu Val Ala Thr Cys Cys Ser Glu Gly Tyr Ala
             20                  25                  30

Leu Pro Ala Val Asn Val Val Gly Thr Asp Ser Ile Asn Ala Val Leu
         35                  40                  45

Glu Ala Ala Ala Arg Asn Arg Ser Asp Val Ile Ile Gln Met Ser Asn
     50                  55                  60

Gly Gly Ala Arg Phe Tyr Ala Gly Glu Gly Met Lys Asp Gln His Arg
 65                  70                  75                  80

Ala Arg Val Leu Gly Ala Val Ala Ala Ala Arg His Val His Thr Leu
                 85                  90                  95

Ala Ala Ala Tyr Gly Val Cys Val Ile Leu His Thr Asp His Ala Asp
            100                 105                 110

Arg Lys Leu Leu Pro Trp Val Ser Asp Leu Ile Asp Glu Ser Glu Ala
        115                 120                 125

Ala Val His Ala Thr Gly Gln Pro Leu Phe Ser Ser His Met Ile Asp
    130                 135                 140

Leu Ser Ala Glu Pro Leu Asp Asp Asn Ile Ala Glu Cys Ala Arg Phe
145                 150                 155                 160

Leu Arg Arg Met Ala Pro Leu Gly Ile Gly Leu Glu Ile Glu Leu Gly
                165                 170                 175

Val Thr Gly Gly Glu Glu Asp Gly Ile Gly His Asp Leu Asp Asp Gly
            180                 185                 190

Ala Asp Asn Ala His Leu Tyr Thr Gln Pro Ala Asp Val Leu Arg Ala
        195                 200                 205

Tyr Asn Glu Leu Ser Pro Leu Gly Phe Val Thr Ile Ala Ala Ser Phe
    210                 215                 220

Gly Asn Val His Gly Val Tyr Ala Pro Gly Asn Val Lys Leu Arg Pro
225                 230                 235                 240

Glu Ile Leu Leu His Ser Gln Gln Ala Val Ser Glu Ala Thr Gly Gln
                245                 250                 255

Gly Glu Arg Pro Leu Ala Leu Val Phe His Gly Gly Ser Gly Ser Glu
            260                 265                 270

Gln Arg Gln Ile Ala Glu Ala Val Ser Tyr Gly Val Phe Lys Met Asn
        275                 280                 285

Ile Asp Thr Asp Ile Gln Phe Ala Phe Ala Glu Gly Val Gly Gly Tyr
    290                 295                 300

Val Leu Glu Asn Pro Glu Ala Phe Arg His Gln Ile Ser Pro Ser Thr
305                 310                 315                 320

Gly Lys Pro Leu Lys Lys Val Tyr Asp Pro Arg Lys Trp Leu Arg Val
                325                 330                 335

Gly Glu Asn Ser Ile Val Ser Arg Leu Asp Gln Thr Phe Ala Asp Leu
            340                 345                 350

Gly Ala Thr Gly Arg Thr Val Ala Ser Ser
        355                 360

<210>    2
<211>    921
<212>    DNA
<213>    Gluconacetobacter xylinus DSM2325 M9

<400>    2
atgacactga caccgcgggt caaggcaatc cttgaccact acgaaagtga cacgccgggc      60

accaaggcca atctctaccg gctcatgaac accggcaagc tcgcgggtac cggcaagctg     120

gtgatcctgc cggttgacca gggcttcgag cacgggccag gccgctcgtt tgcccccaac     180

ccgcccgcct atgacccgca ttatcactac tcgctggcca tcgaggcggg gctgaacgca     240

ttcgcagccc cgctgggcat gcttgaggcc ggggccggca cgtttgccgg ccagatcccc     300

accattctca aatgcaacag ttccaacagc ctgaccacgc agaagaacca ggccgtgacc     360

ggtacggtcg ccgatgcgct gcggctgggc tgctctgcca tcgggtttac catctacccc     420

gccagcgact accagttcca ccagatggag caactgcgcg agatggcacg cgaggccaag     480

aatgcagggc ttgccgtggt ggtgtggagc tacccgcgtg gcccgatgct cgacaaggcg     540

ggcgagacgg ccatcgacat ctgcgcctat gccgcccata tcgccgccga actcggtgcc     600

cacatcatca aggtcaagcc cccgaccgag gatctgtgcc tgcccgcggc caagaaggtc     660

tatatcgatg agaaggtcga tatcgccacc ctgcccgcgc gaatccacca tgtggtgcag     720

tcggcctttg cgggccgccg catagtgatc ttctcgggtg gcgagcacac caccaccgag     780

cacctgctcg acaccattcg cggcatccat caggtggcgg ggttcggttc gatcatcggg     840

cgcaacacct tccagcgccc acgggccgaa gccctgaagc tgcttggtga cattaccgac 900

atcttcctcc agaaggtctg a 921

<210> 3
<211> 1359
<212> DNA
<213> K. xylinus DSM2325 M9

<400> 3
atggtaaaaa caagaaagct gtttggcact gatggcattc ggggcatggc caaccgcttt 60

cccatgacgg tggaagtcgc gcagaagctg ggccaggccg cgggcctgcg cttcatacag 120

ggcacgcacc gccatagcgt gctgctgggc aaggatacgc gcctgtcggg ctatatgatc 180

gaatgcgcgc tggtgtcggg cttcctttcc gccggaatgg acgtgacgct ggtggggccg 240

atgcccaccc cggccattgc catgctcacc cgttccctgc gcgccgatct gggcgtcatg 300

atctcggcgt cgcacaatcc gtatggcgat aacggcatca agctgttcgg ccctgacggg 360

ttcaagctct ccgatgaaac ggaagcgggt attgaagcgg caatgagcga ggacctgacc 420

catatgctcg ccgcccccga ccagatcggc cgggcctcgc gccttaatga cgcggcgggc 480

cggtacgtgg aaagcgccaa gtcctccttc cccgccgcct tgcggcttga cgggctgcgc 540

atcgtaatcg actgcgccaa cggggcggcc tatcgcgtgg cgcccacggc attgtgggaa 600

ctcggtgcgg aagtggtgcg cataggctgc gaccctgatg gcatcaacat caatgaaggc 660

tgcggctcca cccgccccga ggccctgtgt gctgccgtgc agcgccaccg ggccgatatc 720

ggcatcgccc tcgatggcga tgccgaccgc gtgctgattt ctgatgaaaa gggccgcctg 780

atcgatggcg accagatcct ggcgctgatc tcgcattcat gggcgcggca ggggcggctg 840

tcggggcggc atatcgtggc caccgtcatg tccaacatgg ggcttgagcg ctatctcgag 900

acacaggggc tggaactggt gcgcacggcg gtgggcgatc gctacgtggt ggaaaaaatg 960

cgcgagcttg gcgccaatat cggtggcgag cagtcagggc atatggtgct gtcggatttc 1020

gccaccacgg gcgacgggct ggtggcagcc ctgcaggtac tggctgaagt ggtggagtcc 1080

ggtcgccctg caagcgaggt gtgccgcatg ttcaagccct acccgcaact gctgcgcaac 1140

gtgcgctttg ccgggcgcag cccgttgcat gacccgcagg tgcatgacgc gcgcaaggcg 1200

gcggaaaagc ggctgggcgc gcgcgggcga ctcgtgttgc gtgaaagcgg caccgaaccg 1260

ctggtgcgcg tcatggcgga agccgaggac gaagcgctgg tcaatgcggt ggtcgatgac 1320

atgtgcgagg cgattaccgc catccagatg gcgggctga 1359

<210> 4
<211> 452
<212> PRT
<213> K. xylinus DSM2325 M9

<400>    4
Met Val Lys Thr Arg Lys Leu Phe Gly Thr Asp Gly Ile Arg Gly Met
1               5                   10                  15

Ala Asn Arg Phe Pro Met Thr Val Glu Val Ala Gln Lys Leu Gly Gln
            20                  25                  30

Ala Ala Gly Leu Arg Phe Ile Gln Gly Thr His Arg His Ser Val Leu
        35                  40                  45

Leu Gly Lys Asp Thr Arg Leu Ser Gly Tyr Met Ile Glu Cys Ala Leu
    50                  55                  60

Val Ser Gly Phe Leu Ser Ala Gly Met Asp Val Thr Leu Val Gly Pro
65                  70                  75                  80

Met Pro Thr Pro Ala Ile Ala Met Leu Thr Arg Ser Leu Arg Ala Asp
                85                  90                  95

Leu Gly Val Met Ile Ser Ala Ser His Asn Pro Tyr Gly Asp Asn Gly
            100                 105                 110

Ile Lys Leu Phe Gly Pro Asp Gly Phe Lys Leu Ser Asp Glu Thr Glu
        115                 120                 125

Ala Gly Ile Glu Ala Ala Met Ser Glu Asp Leu Thr His Met Leu Ala
    130                 135                 140

Ala Pro Asp Gln Ile Gly Arg Ala Ser Arg Leu Asn Asp Ala Ala Gly
145                 150                 155                 160

Arg Tyr Val Glu Ser Ala Lys Ser Ser Phe Pro Arg Arg Leu Arg Leu
                165                 170                 175

Asp Gly Leu Arg Ile Val Ile Asp Cys Ala Asn Gly Ala Ala Tyr Arg
            180                 185                 190

Val Ala Pro Thr Ala Leu Trp Glu Leu Gly Ala Glu Val Val Arg Ile
        195                 200                 205

Gly Cys Asp Pro Asp Gly Ile Asn Ile Asn Glu Gly Cys Gly Ser Thr
    210                 215                 220

Arg Pro Glu Ala Leu Cys Ala Ala Val Gln Arg His Arg Ala Asp Ile
225                 230                 235                 240

Gly Ile Ala Leu Asp Gly Asp Ala Asp Arg Val Leu Ile Ser Asp Glu
                245                 250                 255

Lys Gly Arg Leu Ile Asp Gly Asp Gln Ile Leu Ala Leu Ile Ser His
            260                 265                 270

Ser Trp Ala Arg Gln Gly Arg Leu Ser Gly Arg His Ile Val Ala Thr
        275                 280                 285

Val Met Ser Asn Met Gly Leu Glu Arg Tyr Leu Glu Thr Gln Gly Leu
    290                 295                 300

Glu Leu Val Arg Thr Ala Val Gly Asp Arg Tyr Val Val Glu Lys Met
305                 310                 315                 320

Arg Glu Leu Gly Ala Asn Ile Gly Gly Glu Gln Ser Gly His Met Val
                325                 330                 335

Leu Ser Asp Phe Ala Thr Thr Gly Asp Gly Leu Val Ala Ala Leu Gln
            340                 345                 350

Val Leu Ala Glu Val Val Glu Ser Gly Arg Pro Ala Ser Glu Val Cys
        355                 360                 365

Arg Met Phe Lys Pro Tyr Pro Gln Leu Leu Arg Asn Val Arg Phe Ala
    370                 375                 380

Gly Arg Ser Pro Leu His Asp Pro Gln Val His Asp Ala Arg Lys Ala
385                 390                 395                 400

Ala Glu Lys Arg Leu Gly Ala Arg Gly Arg Leu Val Leu Arg Glu Ser
                405                 410                 415

Gly Thr Glu Pro Leu Val Arg Val Met Ala Glu Ala Glu Asp Glu Ala
            420                 425                 430

Leu Val Asn Ala Val Val Asp Asp Met Cys Glu Ala Ile Thr Ala Ile
        435                 440                 445

Gln Met Ala Gly
    450

<210>    5
<211>    900
<212>    DNA
<213>    K. xylinus DSM2325 M9

<400>    5
atgagcgaac acggtagcgc aaagccgacc aagggcattc ttctggctgg cgggtcgggc      60

acgcgcctgc accccatgac actggcagtc agcaagcagt tgctgccggt ctatgacaag     120

ccgatgatct tctacccgct ttccacgctc atgctggcgg ggatacgcga tatcatgatc     180

atttccaccc cggccgacct gccgctgttc cgcaggctgc tcggcgatgg ggcggatatg     240

ggtgttacct tcacctaccg cgagcagccc gcgcccgatg gtattgccca ggcttttgtc     300

attgccgatg actggctgga tgattcgccg tgcgggctta ttctgggtga taacctgatc     360

tttgccgacc atctgggcaa gcagatgcgt gcagccgcca cccggccaag cggggccacc     420

gtttttgcct atcaggtgcg tgaccccgag cgttatggcg tggtaagttt tggcgaggac     480

gggcatgcaa tcgatattgt tgaaaaaccc accgaaccca agtcaaactg ggcagtaacg     540

gggctgtatt tttatgatgg tcgcgtgcgt gaatatgcgc gcagcctcag gccctcgccg     600

cgtggcgaac tggaaattac cgacctgaac cgcctttacc tgcagtcgga tgaactgcat     660

gtgcagcgcc ttggccgcgg ctgtgcgtgg cttgatgccg gcatgcccga cagcctgatg     720

caggccgggc agttcgtgca gaccatccag tcccggcagg ggctgctcgt tggctcgccg     780

catgaggtgg ccttccgcat ggggttcatt gatgccgcgg ggcttgaagc ctatgccagg     840

cgcatgatca agaccgaact gggccaggcg ctcatggcca ttgcccatgg cgagggataa     900

                                                                      900

<210>	6
<211>	299
<212>	PRT
<213>	K. xylinus DSM2325 M9

<400>	6
Met Ser Glu His Gly Ser Ala Lys Pro Thr Lys Gly Ile Leu Leu Ala
1               5                   10                  15

Gly Gly Ser Gly Thr Arg Leu His Pro Met Thr Leu Ala Val Ser Lys
            20                  25                  30

Gln Leu Leu Pro Val Tyr Asp Lys Pro Met Ile Phe Tyr Pro Leu Ser
        35                  40                  45

Thr Leu Met Leu Ala Gly Ile Arg Asp Ile Met Ile Ile Ser Thr Pro
    50                  55                  60

Ala Asp Leu Pro Leu Phe Arg Arg Leu Leu Gly Asp Gly Ala Asp Met
65                  70                  75                  80

Gly Val Thr Phe Thr Tyr Arg Glu Gln Pro Ala Pro Asp Gly Ile Ala
                85                  90                  95

Gln Ala Phe Val Ile Ala Asp Asp Trp Leu Asp Asp Ser Pro Cys Gly
            100                 105                 110

Leu Ile Leu Gly Asp Asn Leu Ile Phe Ala Asp His Leu Gly Lys Gln
        115                 120                 125

Met Arg Ala Ala Ala Thr Arg Pro Ser Gly Ala Thr Val Phe Ala Tyr
    130                 135                 140

Gln Val Arg Asp Pro Glu Arg Tyr Gly Val Val Ser Phe Gly Glu Asp
145                 150                 155                 160

Gly His Ala Ile Asp Ile Val Glu Lys Pro Thr Glu Pro Lys Ser Asn
                165                 170                 175

Trp Ala Val Thr Gly Leu Tyr Phe Tyr Asp Gly Arg Val Arg Glu Tyr
            180                 185                 190

Ala Arg Ser Leu Arg Pro Ser Pro Arg Gly Glu Leu Glu Ile Thr Asp
        195                 200                 205

Leu Asn Arg Leu Tyr Leu Gln Ser Asp Glu Leu His Val Gln Arg Leu
    210                 215                 220

Gly Arg Gly Cys Ala Trp Leu Asp Ala Gly Met Pro Asp Ser Leu Met
225                 230                 235                 240

Gln Ala Gly Gln Phe Val Gln Thr Ile Gln Ser Arg Gln Gly Leu Leu
                245                 250                 255

Val Gly Ser Pro His Glu Val Ala Phe Arg Met Gly Phe Ile Asp Ala
            260                 265                 270

Ala Gly Leu Glu Ala Tyr Ala Arg Arg Met Ile Lys Thr Glu Leu Gly
        275                 280                 285

Gln Ala Leu Met Ala Ile Ala His Gly Glu Gly

290                 295

<210>    7
<211>    33
<212>    DNA
<213>    Artificial Sequence

<220>
<223>    primer F1-F

<400>    7
cggcgtagag gatcaggagc ttatcgactg cac                                    33

<210>    8
<211>    28
<212>    DNA
<213>    Artificial Sequence

<220>
<223>    primer F1-R

<400>    8
ccggcgtaga gaatccacag gacgggtg                                          28

<210>    9
<211>    27
<212>    DNA
<213>    Artificial Sequence

<220>
<223>    primer F2-F

<400>    9
ctgtggattc tctacgccgg acgcatc                                           27

<210>    10
<211>    29
<212>    DNA
<213>    Artificial Sequence

<220>
<223>    primer F2-R

<400>    10
aagggcatcg gtcgtcgctc tcccttatg                                         29

<210>    11
<211>    3128
<212>    DNA
<213>    Artificial Sequence

<220>
<223>    pTSa-EX1 vector

<400>    11
gaattcagcc agcaagacag cgatagaggg tagttatcca cgtgaaaccg ctaatgcccc      60

gcaaagcctt gattcacggg gctttccggc ccgctccaaa aactatccac gtgaaatcgc     120

taatcagggt acgtgaaatc gctaatcgga gtacgtgaaa tcgctaataa ggtcacgtga     180

aatcgctaat caaaaaggca cgtgagaacg ctaatagccc tttcagatca acagcttgca     240

aacacccctc gctccggcaa gtagttacag caagtagtat gttcaattag cttttcaatt     300

atgaatatat atatcaatta ttggtcgccc ttggcttgtg gacaatgcgc tacgcgcacc     360

ggctccgccc gtggacaacc gcaagcggtt gcccaccgtc gagcgccagc gcctttgccc     420

acaacccggc ggccggccgc aacagatcgt tttataaatt tttttttttg aaaaagaaaa     480

agcccgaaag gcggcaacct ctcgggcttc tggatttccg atcacctgta agtcggacgc     540

gatgcgtccg gcgtagagga tccggagctt atcgactgca cggtgcacca atgcttctgg     600

cgtcaggcag ccatcggaag ctgtggtatg gctgtgcagg tcgtaaatca ctgcataatt     660

cgtgtcgctc aaggcgcact cccgttctgg ataatgtttt ttgcgccgac atcataacgg     720

ttctggcaaa tattctgaaa tgagctgttg acaattaatc atcggctcgt ataatgtgtg     780

gaattgtgag cggataacaa tttcacacag ggacgagcta ttgattgggt accgagctcg     840

aattcgtacc cggggatcct ctagagtcga cctgcaggca tgcaagcttg gctgttttgg     900

cggatgagag aagattttca gcctgataca gattaaatca gaacgcagaa gcggtctgat     960

aaaacagaat ttgcctggcg gcagtagcgc ggtggtccca cctgacccca tgccgaactc    1020

agaagtgaaa cgccgtagcg ccgatggtag tgtggggtct ccccatgcga gagtagggaa    1080

ctgccaggca tcaaataaaa cgaaaggctc agtcgaaaga ctgggccttt cgttttatct    1140

gttgtttgtc ggtgaacgct ctcctgagta ggacaaatcc gccgggagcg gatttgaacg    1200

ttgcgaagca acggcccgga gggtggcggg caggacgccc gccataaact gccaggcatc    1260

aaattaagca gaaggccatc ctgacggatg gcctttttgc cttccgcttc ctcgctcact    1320

gactcgctgc gctcggtcgt tcggctgcgg cgagcggtat cagctcactc aaaggcggta    1380

atacggttat ccacagaatc aggggataac gcaggaaaga acatgtgagc aaaaggccag    1440

caaaaggcca ggaaccgtaa aaaggccgcg ttgctggcgt ttttccatag gctccgcccc    1500

cctgacgagc atcacaaaaa tcgacgctca agtcagaggt ggcgaaaccc gacaggacta    1560

taaagatacc aggcgtttcc ccctggaagc tccctcgtgc gctctcctgt tccgaccctg    1620

ccgcttaccg gatacctgtc cgcctttctc ccttcgggaa gcgtggcgct ttctcatagc    1680

tcacgctgta ggtatctcag ttcggtgtag gtcgttcgct ccaagctggg ctgtgtgcac    1740

gaaccccccg ttcagcccga ccgctgcgcc ttatccggta actatcgtct tgagtccaac    1800

ccggtaagac acgacttatc gccactggca gcagccactg gtaacaggat tagcagagcg    1860

aggtatgtag gcggtgctac agagttcttg aagtggtggc ctaactacgg ctacactaga 1920

agaacagcat ttggtatctg cgctctgctg aagccagtta ccttcggaaa aagagttggt 1980

agctcttgat ccggcaaaca aaccaccgct ggtagcggtg gtttttttgt ttgcaagcag 2040

cagattacgc gcagaaaaaa aggatctcaa gaagatcctt tgatcttttc tacggggtct 2100

gacgctcagt ggaacgaaaa ctcacgttaa aggctgtgca ggtcgtaaat cactgcataa 2160

ttcgtgtcgc tcaaggcgca ctcccgttct ggataatgtt ttttgcgccg acatcataac 2220

ggttctggca aatattctga aatgagctgt tgacaattaa tcatcggctc gtataatgtg 2280

tggaattgtg agcggataac aatttcacac aggaaacata gatctcccgg gtaccgagct 2340

ctctagaaag aaggagggac gagctattga tggagaaaaa aatcactgga tataccaccg 2400

ttgatatatc ccaatggcat cgtaaagaac attttgaggc atttcagtca gttgctcaat 2460

gtacctataa ccagaccgtt cagctggata ttacggcctt tttaaagacc gtaaagaaaa 2520

ataagcacaa gttttatccg gcctttattc acattcttgc ccgcctgatg aatgctcatc 2580

cggaattccg tatggcaatg aaagacggtg agctggtgat atgggatagt gttcaccctt 2640

gttacaccgt tttccatgag caaactgaaa cgttttcatc gctctggagt gaataccacg 2700

acgatttccg gcagtttcta cacatatatt cgcaagatgt ggcgtgttac ggtgaaaacc 2760

tggcctattt ccctaaaggg tttattgaga atatgttttt cgtctcagcc aatccctggg 2820

tgagtttcac cagttttgat ttaaacgtgg ccaatatgga caacttcttc gcccccgttt 2880

tcaccatggg caaatattat acgcaaggcg acaaggtgct gatgccgctg gcgattcagg 2940

ttcatcatgc cgtttgtgat ggcttccatg tcggcagaat gcttaatgaa ttacaacagt 3000

actgcgatga gtggcagggc ggggcgtaat ttttttaagg cagttattta aggcagttat 3060

tggtgccctt aaacgcctgg ttgctacgcc tgaataagtg ataataagcg gatgaatggc 3120

agaaattc 3128

<210> 12
<211> 32
<212> DNA
<213> Artificial Sequence

<220>
<223> primer GX1979_F

<400> 12
tcctctagag tcgacatgac actgacaccg cg 32

<210> 13
<211> 37
<212> DNA
<213> Artificial Sequence

<220>
<223>    primer GX1979_R


<400>    13
tgcctgcagg tcgactcaga ccttctggag gaagatg                                37


<210>    14
<211>    40
<212>    DNA
<213>    Artificial Sequence

<220>
<223>    primer gx1215-F


<400>    14
tcctctagag tcgacatggt aaaaacaaga aagctgtttg                             40


<210>    15
<211>    34
<212>    DNA
<213>    Artificial Sequence

<220>
<223>    primer gx1215-R


<400>    15
tgcctgcagg tcgactcagc ccgccatctg gatg                                   34


<210>    16
<211>    33
<212>    DNA
<213>    Artificial Sequence

<220>
<223>    primer gx2556-F


<400>    16
tcctctagag tcgacatgag cgaacacggt agc                                    33


<210>    17
<211>    33
<212>    DNA
<213>    Artificial Sequence

<220>
<223>    primer gx2556-R


<400>    17
tgcctgcagg tcgacttatc cctcgccatg ggc                                    33


<210>    18
<211>    2166

<212>     DNA
<213>     K.xylinus

<400>     18
atgatctggc gcattttaaa atctcccctc gtctccggcc cgttattcgc catcctcctg          60

gcagtggtct gcctgaccta cctctccccc gaccaccagt ttttcgtcgc gatagggggc          120

gcgatcctgt tctttctggt tcgccgacat gatgaacgct ggtcgcgctg ttttctcatg          180

gtgctgtcca tcgtggtatc cgggcgctat ctggtgtggc gctttacctc cacgcttgat          240

ctcgatggcg tgttgcagac agttctagtc ctggcgctgg cgatcggcga aatctatacc          300

accttccggg tgggctttac gtatttccag ttggcctggc ccctgcggcg gcagatccac          360

ccgctgccgg aagatgaagg cagttggccg gtcattgatg tctatgtgcc aacctataac          420

gaggacatgg cgatcgtccg caccacggtg ctgggctgcc tggccatgga ctggccggca          480

gacaagctga atgtctatat ccttgatgac gggcggcggc gctcgttccg tgattttgcc          540

gcgcaggtcg gtgctggcta catcaatcgc gcggacagta cccacgccaa ggcgggcaac          600

ctcaaccatg ccatcaaggc gacaacgggc gacctgatcg cgatctttga ctgtgaccat          660

gtgcccgtgc ggggtttcct caaaaagacc gtggggtgga tgatcgccga ccccaacctc          720

gcgctattgc agaccccgca tcacttctat tcccccgatc cgttccgtcg caacatgagc          780

cggggcatgc aggtgccgcc cgagagcaac ctgttctatg ggcttttgca agatggcaat          840

gatttctgga acgccacctt cttctgcggg tcgtgcgccc tgctgcggcg cgaggccatt          900

gaagcgatca atggctttgc cgtcgagacc gtgacggaag atgcccacac cgccctgcgc          960

atgcagcgca aggggtgggg cacggcctat ctgcgcgagc cgctggccgc ggggctcgaa          1020

accgaaaccc tcctgcttca ggtcgggcag cgcgtgcgct gggcgcgcgg catgatccag          1080

atgctgcggc tcgacaaccc catgctcggc cgtggcctgc gcctcacgca gcgtatctgc          1140

tacatggcgg cgacgacgaa ctacttcttc gccatgccgc gcatcatgtt cctcatggcg          1200

ccgctggcct acctgttcct gggcgtgacc atgatcgcgg cctcgcctta tgaacttgcg          1260

gtctatgccc tgccgcacct gtttcatacc accatgacca tgtcgcgcct gcaggggcgg          1320

tggcgctatt cgttctggag cgagatctac gaatccatgc tggccccctt tctggtgcgc          1380

atgacgttca tcaccctgct tgcgccgcac aagggcaagt tcaacgtgac cgacaagggc          1440

ggcctgctgc accgcgagta ttttgaatgg cgcgcggcct accccggcgt gatcatggcc          1500

gtggtgctgg cggtgggact ggtgagcggc atctgggccg cgattgcccc ttatcatgaa          1560

acgctcgtct tccgcgccat ggcggtcaac tcggtctggg tgctgttcag cctgatcatc          1620

gtgcttggtg gtgtggccgc cgcgcgcgaa acccgccagc gccgccgtaa ccaccgcgtt          1680

gcggccagca ttcccctgac catgttcacg ggtgatacgc aggtcaccgc ctgtcgcacg          1740

ctggatgtgt cgatgggggg ctgccagctt gacctgtcgc ccacactgcc ccttgccgtg          1800

ggggatgaac tgcgcctgca cgccaccctg gcctccggcc cgatcacgct ccgcgccacc 1860

ctcatcgacc ggcatgaggg ccgtgcccat gtggcgtgga tcatgcccga cctcgcggcc 1920

gagaagcagg tcgtggccct ggtgtttggc cgtgatgatg cctggtccca gtggtccgac 1980

ttcccgcctg acaggccgct tcacagtctt tacatgctgc ttgccagcat ctgcgcgctg 2040

ttccgcccct atccgcgcgg gcagtcggat gcgccgccac cgcccgcgcc gcctcccccg 2100

atcgcagagg aaaaactgcc ggcacggcat ctggttatac caaccgttga ttgctataac 2160

ctatga 2166

<210> 19
<211> 721
<212> PRT
<213> K.xylinus

<400> 19
Met Ile Trp Arg Ile Leu Lys Ser Pro Leu Val Ser Gly Pro Leu Phe
1               5                   10                  15

Ala Ile Leu Leu Ala Val Val Cys Leu Thr Tyr Leu Ser Pro Asp His
            20                  25                  30

Gln Phe Phe Val Ala Ile Gly Gly Ala Ile Leu Phe Phe Leu Val Arg
        35                  40                  45

Arg His Asp Glu Arg Trp Ser Arg Cys Phe Leu Met Val Leu Ser Ile
    50                  55                  60

Val Val Ser Gly Arg Tyr Leu Val Trp Arg Phe Thr Ser Thr Leu Asp
65                  70                  75                  80

Leu Asp Gly Val Leu Gln Thr Val Leu Val Leu Ala Leu Ala Ile Gly
                85                  90                  95

Glu Ile Tyr Thr Thr Phe Arg Val Gly Phe Thr Tyr Phe Gln Leu Ala
            100                 105                 110

Trp Pro Leu Arg Arg Gln Ile His Pro Leu Pro Glu Asp Glu Gly Ser
        115                 120                 125

Trp Pro Val Ile Asp Val Tyr Val Pro Thr Tyr Asn Glu Asp Met Ala
    130                 135                 140

Ile Val Arg Thr Thr Val Leu Gly Cys Leu Ala Met Asp Trp Pro Ala
145                 150                 155                 160

Asp Lys Leu Asn Val Tyr Ile Leu Asp Asp Gly Arg Arg Arg Ser Phe
                165                 170                 175

Arg Asp Phe Ala Ala Gln Val Gly Ala Gly Tyr Ile Asn Arg Ala Asp
            180                 185                 190

Ser Thr His Ala Lys Ala Gly Asn Leu Asn His Ala Ile Lys Ala Thr
        195                 200                 205

Thr Gly Asp Leu Ile Ala Ile Phe Asp Cys Asp His Val Pro Val Arg
    210                 215                 220

Gly Phe Leu Lys Lys Thr Val Gly Trp Met Ile Ala Asp Pro Asn Leu
225                 230                 235                 240

Ala Leu Leu Gln Thr Pro His His Phe Tyr Ser Pro Asp Pro Phe Arg
                245                 250                 255

Arg Asn Met Ser Arg Gly Met Gln Val Pro Pro Glu Ser Asn Leu Phe
            260                 265                 270

Tyr Gly Leu Leu Gln Asp Gly Asn Asp Phe Trp Asn Ala Thr Phe Phe
        275                 280                 285

Cys Gly Ser Cys Ala Leu Leu Arg Arg Glu Ala Ile Glu Ala Ile Asn
    290                 295                 300

Gly Phe Ala Val Glu Thr Val Thr Glu Asp Ala His Thr Ala Leu Arg
305                 310                 315                 320

Met Gln Arg Lys Gly Trp Gly Thr Ala Tyr Leu Arg Glu Pro Leu Ala
                325                 330                 335

Ala Gly Leu Glu Thr Glu Thr Leu Leu Leu Gln Val Gly Gln Arg Val
            340                 345                 350

Arg Trp Ala Arg Gly Met Ile Gln Met Leu Arg Leu Asp Asn Pro Met
        355                 360                 365

Leu Gly Arg Gly Leu Arg Leu Thr Gln Arg Ile Cys Tyr Met Ala Ala
    370                 375                 380

Thr Thr Asn Tyr Phe Phe Ala Met Pro Arg Ile Met Phe Leu Met Ala
385                 390                 395                 400

Pro Leu Ala Tyr Leu Phe Leu Gly Val Thr Met Ile Ala Ala Ser Pro
                405                 410                 415

Tyr Glu Leu Ala Val Tyr Ala Leu Pro His Leu Phe His Thr Thr Met
            420                 425                 430

Thr Met Ser Arg Leu Gln Gly Arg Trp Arg Tyr Ser Phe Trp Ser Glu
        435                 440                 445

Ile Tyr Glu Ser Met Leu Ala Pro Phe Leu Val Arg Met Thr Phe Ile
    450                 455                 460

Thr Leu Leu Ala Pro His Lys Gly Lys Phe Asn Val Thr Asp Lys Gly
465                 470                 475                 480

Gly Leu Leu His Arg Glu Tyr Phe Glu Trp Arg Ala Ala Tyr Pro Gly
                485                 490                 495

Val Ile Met Ala Val Val Leu Ala Val Gly Leu Val Ser Gly Ile Trp
            500                 505                 510

Ala Ala Ile Ala Pro Tyr His Glu Thr Leu Val Phe Arg Ala Met Ala
        515                 520                 525

Val Asn Ser Val Trp Val Leu Phe Ser Leu Ile Ile Val Leu Gly Gly
    530                 535                 540

Val Ala Ala Ala Arg Glu Thr Arg Gln Arg Arg Arg Asn His Arg Val
545                 550                 555                 560

Ala Ala Ser Ile Pro Leu Thr Met Phe Thr Gly Asp Thr Gln Val Thr
                565                 570                 575

Ala Cys Arg Thr Leu Asp Val Ser Met Gly Gly Cys Gln Leu Asp Leu
            580                 585                 590

Ser Pro Thr Leu Pro Leu Ala Val Gly Asp Glu Leu Arg Leu His Ala
        595                 600                 605

Thr Leu Ala Ser Gly Pro Ile Thr Leu Arg Ala Thr Leu Ile Asp Arg
    610                 615                 620

His Glu Gly Arg Ala His Val Ala Trp Ile Met Pro Asp Leu Ala Ala
625                 630                 635                 640

Glu Lys Gln Val Val Ala Leu Val Phe Gly Arg Asp Asp Ala Trp Ser
                645                 650                 655

Gln Trp Ser Asp Phe Pro Pro Asp Arg Pro Leu His Ser Leu Tyr Met
            660                 665                 670

Leu Leu Ala Ser Ile Cys Ala Leu Phe Arg Pro Tyr Pro Arg Gly Gln
        675                 680                 685

Ser Asp Ala Pro Pro Pro Pro Ala Pro Pro Pro Pro Ile Ala Glu Glu
    690                 695                 700

Lys Leu Pro Ala Arg His Leu Val Ile Pro Thr Val Asp Cys Tyr Asn
705                 710                 715                 720

Leu

## Description

### BACKGROUND

### 1. Field

The present disclosure relates to a microorganism of the genus *Gluconacetobacter* having cellulose productivity, a method of producing cellulose using the same, and a method of producing the microorganism.

### 2. Description of the Related Art

Cellulose can be harvested from cultures of a microorganism. The so-produced cellulose is primarily composed of glucose in the form of β-1,4 glucan units. On a larger scale, cellulose molecules form a network structure of fibril bundles. This cellulose is also called 'bio-cellulose or microbial cellulose'.

Unlike plant cellulose, microbial cellulose is pure cellulose entirely free of lignin or hemicellulose. Microbial cellulose, which is typically 100 nm or less in width, has network structure of bundles of cellulose nanofibers, and has characteristic properties such as high water absorption and retention capacity, high tensile strength, high elasticity, and high heat resistance, compared to plant cellulose. They make it suitable for use in a variety of fields, including cosmetics, medical products, dietary fibers, audio speaker diaphragms, functional films, etc.

*Acetobacter, Agrobacteria, Rhizobia,* and *Sarcina* have been reported as microbial cellulose-producing strains. Upon static culture under aerobic conditions, cellulose with a three-dimensional network structure is formed as a thin film on the surface of a culture of these microorganisms.

Still, there is a demand for a recombinant microorganism of the genus *Gluconacetobacter* having cellulose productivity.

### SUMMARY

An aspect provides a microorganism of the genus *Gluconacetobacter* having cellulose productivity. The recombinant microorganism comprises a genetic modification that increases fructose-bisphosphate aldolase (FBA) activity.

Another aspect provides a method of producing cellulose using the microorganism. The method comprises culturing a recombinant microorganism of the genus *Gluconacetobacter* having cellulose productivity in a culture medium and collecting cellulose from the culture medium, wherein the microorganism comprising a genetic modification that increases fructose-bisphosphate aldolase activity.

Also provided is a method of producing the recombinant microorganism having cellulose productivity, the method comprising introducing a genetic modification that increases fructose-bisphosphate aldolase (FBA) activity into a microorganism of the genus *Gluconacetobacter.*

### DETAILED DESCRIPTION

The term "parent cell" refers to a cell in a state immediately prior to a particular genetic modification, for example, a cell that serves as a starting material for producing a cell having a genetic modification that increases or decreases the activity of one or more proteins. A parent cell, thus, is a cell without a particular referenced genetic modification, but with the other genotypic and phenotypic traits of the genetically modified cell. Although the "parent cell" does not have the specific referenced genetic modification, the parent cell may be engineered in other respects and, thus, might not be a "wild-type" cell (though it may also be a wild-type cell if no other modifications are present). Thus, the parent cell may be a cell used as a starting material to produce a genetically engineered microorganism having an inactivated or decreased activity of a given protein (e.g., a protein having a sequence identity of about 95% or more to a protein having a sequence identity of about 95% or more with respect to fructose-bisphosphate aldolase or other protein) or a genetically engineered microorganism having an increased activity of a given protein (e.g., a protein having a sequence identity of about 95% or more to the protein). By way of further illustration, with respect to a cell in which a gene encoding a protein has been modified to reduce gene activity, the parent cell may be a microorganism including an unaltered, "wild-type" gene. The same comparison is applied to other genetic modifications.

The recombinant microorganism may have an enhanced cellulose productivity compared to a parent microorganism. The term "enhanced cellulose productivity", as used herein, refers to a detectable increase in cellulose production compared to a cellulose production from a parent cell. The cellulose production may be measured by weighing the weight of produced cellulose. The weight may be a weight of dried cellulose.

The term "increase in activity" or "increased activity", as used herein, refers to a detectable increase in an activity of a cell, a protein, or an enzyme including a modified (e.g., genetically engineered) cell, protein, or enzyme that is higher than that of a comparative (e.g., parent, wild-type, or control) cell, protein, or enzyme of the same type, such as a cell, protein, or enzyme that does not have a given genetic modification (e.g., original or "wild-type" cell, protein, or enzyme). "Cell activity" refers to an activity of a particular protein or enzyme of a cell. For example, an activity of a modified or engineered cell, protein, or enzyme can be increased by about 5% or more, about 10% or more, about 15% or more, about 20% or more, about 30% or more, about 50% or more, about 60% or more, about 70% or more, or about 100% or more than an activity of a non-engineered cell, protein, or enzyme of the same type, i.e., a wild-type cell, protein, or enzyme. A cell having an increased activity of a protein or an enzyme can be identified by using any method known in the art.

An increase in activity of an enzyme or a polypeptide can be achieved by an increase in the expression or specific activity thereof. The increase in the expression can be achieved by introduction of an exogenous gene encoding the enzyme or the polypeptide into a cell, by an increase in a copy number of a gene encoding the enzyme or polypeptide in a cell, or by a mutation (including point mutations and promoter-swaps) in the regulatory region of an endogenous polynucleotide. The microorganism receiving the exogenous gene may already contain a copy of the gene endogenously, or might not include the gene prior to its introduction. The gene may be operably linked to a regulatory sequence that allows expression thereof, for example, a promoter, an enhancer, a polyadenylation region, or a combination thereof (e.g., by inclusion in an expression cassette of an appropriate vector). An exogenous gene refers to a gene introduced into a cell from the outside. The introduced exogenous gene may be endogenous or heterologous with respect to the host cell. An endogenous gene refers to a gene that already exists in the genetic material of a microorganism (e.g., a native gene). A heterologous gene is one that does not normally exist in the genetic material of a given microorganism (e.g., foreign or not native).

An increase in the copy number can be caused by introduction of an exogenous gene or by amplification of an endogenous gene, and encompasses genetically engineering a cell so that the cell has a gene that does not exist in a non-engineered cell (i.e., introduction of an exogenous heterologous gene, thereby increasing copy number from 0 to 1). The introduction of the gene can be mediated by a vehicle such as a vector. The introduction can result in a gene that is not integrated into a genome (e.g. a transient or stable episome such as a plasmid or artificial chromosome), or an integration of the gene into the genome (e.g., by homologous recombination). The introduction can be performed, for example, by introducing a vector into the cell, the vector including a polynucleotide encoding a target polypeptide, and then replicating the vector in the cell or by integrating the polynucleotide into the genome.

The introduction of the gene may be performed by a known method, such as transformation, transfection, and electroporation. The gene may be introduced via a vehicle or by itself. As used herein, the term "vehicle" refers to a nucleic acid molecule capable of delivering other nucleic acids linked thereto (e.g., a vector, a nucleic acid construct, or a cassette). Examples of a vector include a plasmid vector, a virus-derived vector, etc. A plasmid (e.g., plasmid expression vector) is a circular double-stranded DNA molecule linkable with other DNA. Examples of viral expression vectors include replication-defective retrovirus, adenovirus, adeno-associated virus, and the like.

The term "gene", as used herein, refers to a nucleic acid fragment expressing a specific protein, and the fragment may or may not include one or more regulatory sequences (e.g., 5'-non coding sequence and/or 3'-non coding sequence).

"Sequence identity" of a nucleic acid or a polypeptide, as used herein, refers to the extent of identity between nucleotides or amino acid residues of sequences obtained after the sequences are aligned so as to best match in certain comparable regions. Sequence identity is a value obtained by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear to obtain the number of matching locations, dividing the number of matching locations by the total number of locations in the comparable regions (e.g., the size of a range), and multiplying a result of the division by 100 to obtain the percentage of the sequence identity. The percentage of the sequence identity may be determined using a known sequence comparison program, for example, BLASTN (NCBI), BLASTP (NCBI), CLC Main Workbench (CLC bio), or MegAlign™ (DNASTAR Inc), or Needleman-Wunsch global alignment algorithm (e.g., EMBOSS Needle). Unless otherwise specified, selection of parameters used for operating the program is as follows: Ktuple=2, Gap Penalty=4, and Gap length penalty=12. Sequence identity comparisons can also be performed manually, where optimal alignment is clearly ascertained.

Various levels of sequence identity may be used to identify various types of polypeptides or polynucleotides having the same or similar functions or activities. For example, the sequence identity may include a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100%.

Where a polynucleotide sequence encoding a given protein, other polynucleotide sequences can be substituted due to the degeneracy of the genetic code.

The term "genetic modification", as used herein, includes an artificial alteration in a constitution or structure of genetic material of a cell, which can be accomplished using any suitable technique.

In the present invention, unless otherwise specified, % represents w/w%.

An aspect provides a recombinant microorganism of the genus *Gluconacetobacter* having cellulose productivity, the microorganism including a genetic modification that increases the activity of fructose-bisphosphate aldolase (FBA).

Fructose-bisphosphate aldolase (FBA) catalyzes the reaction: fructose-1,6-bisphosphate(FBP) ⇔ dihydroxyacetone phosphate(DHAP) + glyceraldehyde 3-phosphate(G3P). The fructose-bisphosphate aldolase can belong to EC 4.1.2.13. The fructose-bisphosphate aldolase can be exogenous or endogenous. The fructose-bisphosphate aldolase can be in the form of a monomer consisting of a single polypeptide. The fructose-bisphosphate aldolase can be selected from the group consisting of fructose-bisphosphate aldolases derived from the genus *Gluconacetobacter,* the genus *Bacillus,* the genus *Mycobacterium,* the genus *Zymomonas,* the genus *Vibrio,* and the genus *Escherichia.*

The fructose-bisphosphate aldolase can be a polypeptide having a sequence identity of about 95% or more with respect to an amino acid sequence of SEQ ID NO: 1.

In the microorganism, the genetic modification can increase expression of a gene encoding fructose-bisphosphate aldolase. For instance, the genetic modification can be an increased copy number of the fructose-bisphosphate aldolase gene (e.g., a gene encoding the polypeptide having a sequence identity of about 95% or more with respect to the amino acid sequence of SEQ ID NO: 1). For example, the gene can have a nucleotide sequence of SEQ ID NO: 2, or a sequence having at least 85%, 90%, or 95% sequence identity to the sequence of SEQ ID NO: 2.

In one aspect, the genetic modification may be the introduction of a gene encoding fructose-bisphosphate aldolase, for example, via a vehicle such as a vector. The fructose-bisphosphate aldolase and gene encoding same that is introduced into the microorganism may be endogenous or heterologous. The gene encoding fructose-bisphosphate aldolase once introduced may integrate within the host genome or remain independent (outside) of the chromosome. Furthermore, the genetic modification may include the introduction of a plurality of genes encoding fructose-bisphosphate aldolase, for example, 2 or more, 5 or more, 10 or more, 50 or more, 100 or more, or 1000 or more genes, which may be the same (e.g., multiple copies of a gene) or different provided they encode fructose bisphosphate aldolase.

The microorganism can be of the genus *Gluconacetobacter,* for example, *G. aggeris, G. asukensis, G. azotocaptans, G. diazotrophicus, G. entanii, G. europaeus, G. hansenii, G. intermedius, G. johannae, G. kakiaceti, G. kombuchae, G. liquefaciens, G. maltaceti, G. medellinensis, G. nataicola, G. oboediens, G. rhaeticus, G. sacchari, G. saccharivorans, G. sucrofermentans, G. swingsii, G. takamatsuzukensis, G. tumulicola, G. tumulisoli,* or *G. xylinus* (also called *"Komagataeibacterxylinus"* or *"K. xylinus*")*.*

The microorganism can further include one or more genetic modifications selected from the group consisting of a genetic modification that increases activity of phosphoglucomutase (PGM) which catalyzes conversion of glucose-6-phosphate to glucose-1-phosphate, a genetic modification that increases activity of UTP-glucose-1-phosphate uridylyltransferase (UGP) which catalyzes conversion of glucose-1-phosphate to UDP-glucose, and a genetic modification that increases activity of cellulose synthase (CS) which catalyzes conversion of UDP-glucose to cellulose. The genetic modification may be an increase in the copy number of the above one or more genes. PGM may belong to EC 2.7.7.9, and UGP may belong to EC 5.4.2.2 or EC 5.4.2.5.

The polypeptide having PGM activity can be, for instance, a polypeptide having a sequence identity of about 95% or more with respect to an amino acid sequence of SEQ ID NO: 4. Similarly, the polypeptide having UPG activity can be a polypeptide having a sequence identity of about 95% or more with respect to an amino acid sequence of SEQ ID NO: 6, and the polypeptide having CS activity can be a polypeptide having a sequence identity of about 95% or more with respect to an amino acid sequence of SEQ ID NO: 19.

In an embodiment, the microorganism may have an increase in the copy number of one or more genes selected from the group consisting of a gene having a nucleotide sequence of SEQ ID NO: 3 (or a sequence having at least 85%, 90%, or 95% sequence identity to the sequence of SEQ ID NO: 3), a gene having a nucleotide sequence of SEQ ID NO: 5 (or a sequence having at least 85%, 90%, or 95% sequence identity to the sequence of SEQ ID NO: 5), and a gene having a nucleotide sequence of SEQ ID NO: 18 (or a sequence having at least 85%, 90%, or 95% sequence identity to the sequence of SEQ ID NO: 18).

Another aspect provides a method of producing cellulose. The method includes culturing the recombinant microorganism of the genus *Gluconacetobacter* having cellulose productivity. The microorganism includes a genetic modification that increases the activity of fructose-bisphosphate aldolase. The microorganism is cultured in a medium to produce cellulose; and the cellulose is obtained (isolated or otherwise collected) from the culture.

The culturing may be performed in a medium containing a suitable carbon source, for example, glucose. The medium used for culturing the microorganism may be any general medium suitable for host cell growth, such as a minimal or complex medium containing appropriate supplements. The suitable medium may be commercially available or prepared by a known preparation method.

The medium may be a medium that may satisfy the requirements of the particular microorganism used. The medium may be a medium including components selected from the group consisting of a carbon source, a nitrogen source, a salt, trace elements, and combinations thereof. The medium may include ethanol of about 0.5 % to about 3%(v/v), for example, about 0.5 % to about 2.5%(v/v), about 0.75 % to about 2.25%(v/v), or about 1.0 % to about 2.0 %(v/v).

The culturing conditions may be appropriately controlled for the production of cellulose. The culturing may be performed under aerobic conditions for cell proliferation. The culturing may be performed by static culture without shaking. The culturing may be performed with a low density of the microorganism. The density of the microorganism may be a density which provides intercellular space sufficient to not to disturb secretion of cellulose.

The term "culture conditions", as used herein, mean conditions for culturing the microorganism. Such culture conditions may include, for example, a carbon source, a nitrogen source, or an oxygen condition utilized by the microorganism. The carbon source that may be utilized by the microorganism may include monosaccharides, disaccharides, or polysaccharides. The carbon source may include glucose, fructose, mannose, or galactose as an assimilable sugar. The nitrogen source may be an organic nitrogen compound or an inorganic nitrogen compound. The nitrogen source may be exemplified by amino acids, amides, amines, nitrates, or ammonium salts. An oxygen condition for culturing the microorganism may be an aerobic condition of a normal oxygen partial pressure, or a low-oxygen condition including about 0.1% to about 10% of oxygen in the atmosphere. A metabolic pathway may be modified in accordance with a carbon source or a nitrogen source that may be actually used by a microorganism.

The method may include separating and collecting the cellulose from the culture. Separation may be accomplished, for example, by collecting a cellulose pellicle formed on the top of the medium. The cellulose pellicle may be collected by physically stripping off the cellulose pellicle or by removing the medium. The separation may involve collecting the cellulose pellicle while maintaining its shape without damage.

Still another aspect provides a method of producing the microorganism having cellulose productivity. The method includes introducing a genetic modification that increases fructose bisphosphate aldolase (FBA) activity into the microorganism of the genus *Gluconacetobacter.* The genetic modification may be any described herein with respect to the recombinant microorganism. Thus, for instance, the genetic modification can be introduction of an exogenous gene encoding fructose bisphosphate aldolase into the microorganism of the genus *Gluconacetobacter.* The gene may be heterologous or endogenous. The introducing of the gene encoding fructose-bisphosphate aldolase can be achieved by introducing a vehicle including the gene into the microorganism. In addition or instead, the genetic modification can include amplification of an endogenous gene, manipulation of the regulatory sequence of the gene, or manipulation of the sequence of the gene itself, such as by insertion, substitution, conversion, or addition of nucleotides.

The method may further include introducing one or more genetic modifications selected from the group consisting of a genetic modification that increases activity of PGM that catalyzes conversion of glucose-6-phosphate to glucose-1-phosphate, a genetic modification that increases activity of UPG that catalyzes conversion of glucose-1-phosphate to UDP-glucose, and a genetic modification that increases activity of cellulose synthase that catalyzes conversion of UDP-glucose to cellulose, as previously described herein. For instance, the genetic modification may increase the copy number of one or more genes selected from the group consisting of a gene encoding a polypeptide having PGM activity, the polypeptide having a sequence identity of about 95% or more with respect to the amino acid sequence of SEQ ID NO: 4 and a gene encoding a polypeptide having UPG activity, the polypeptide having a sequence identity of about 95% or more with respect to the amino acid sequence of SEQ ID NO: 6. The introducing of the genetic modification may be achieved by increasing the copy number of one or more genes selected from the group consisting of a gene having a nucleotide sequence of SEQ ID NO: 3 and a gene having a nucleotide sequence of SEQ ID NO: 5.

The recombinant microorganism of the genus *Gluconacetobacter* having cellulose productivity may be used to produce cellulose efficiently and/or in a high yield.

Reference will now be made in detail to embodiments. In this regard, the present embodiments may have different forms and should not be construed as being limited to the descriptions set forth herein. As used herein, the term "and/or" includes any and all combinations of one or more of the associated listed items.

In accordance with an embodiment, a method of producing the recombinant organism with cellulose efficiency comprises making the genetic modifications described above or in the Examples below.

### Example 1. Preparation of K.xylinus including Fructose-bisphosphate aldolase gene and Production of Cellulose

In this Example, *K.xylinus* DSM2325 was transformed with an exogenous or endogenous fructose-bisphosphate aldolase (FBA) gene. The resulting microorganism cultured to produce cellulose to examine the effects of the gene introduction on cellulose productivity.

### 1. Introduction of fructose-bisphosphate aldolase gene

A *K. xylinus* DSM2325 GX_1979 gene with a nucleotide sequence of SEQ ID NO: 2 was introduced into a *K.xylinus* DSM2325 M9 strain. The specific introduction procedures performed are as follows.

### (1) Construction of vector

PCR was performed using a pTSa-EX1 vector (SEQ ID NO: 11) as a template and a set of primers of either F1-F (SEQ ID NO: 7) and F1-R (SEQ ID NO: 8) or F2-F (SEQ ID NO: 9) and F2-R (SEQ ID NO: 10) to obtain a PCR product of 1.9 kb or 0.3 kb, respectively. The PCR product had a point mutation in the sequence of the vector, and one restriction enzyme site was removed therefrom. This PCR product was cloned into BamHI/Sall restriction sites of the pTSa-EX1 vector using an In-Fusion GD cloning kit (Takara) to prepare a pTSa-EX11 vector.

Next, an open reading frame (ORF) of a fructose-bisphosphate aldolase GX_1979 gene was obtained by gel extraction after PCR using genomic DNA of the K.xylinus DSM2325 M9 strain as a template and a set of primers of SEQ ID NO: 12 and SEQ ID NO: 13.

The ORF of the gene was cloned into a Sall restriction site of the pTSa-EX11 vector using an In-Fusion GD cloning kit (Takara) to prepare an overexpression vector pTSa-GX1979.

### (2) Transformation

The *K. xylinus* DSM2325 M9 strain was spread on a plate containing an HS-agar medium supplemented with 2% glucose, and cultured at 30°C for 3 days. The strain thus cultured was flushed with 2 ml of sterile water, and colonies were pooled. The colonies were transferred to a 50 ml falcon tube, followed by vortexing for 2 minutes. The 2% glucose-containing HS-agar medium included 0.5% peptone, 0.5% yeast extract, 0.27% Na₂HPO₄, 0.15% citric acid, 2% glucose, and 1.5% bacter-agar. Thereafter, 1% cellulase (sigma, Cellulase from Trichoderma reesei ATCC 26921) was added and allowed to react at 30°C and 160 rpm for 2 hours. Then, the colonies were washed with 1 mM HEPES buffer-containing medium and then washed with 15(w/w)% glycerol three times, followed by resuspension in 1 ml of 15(w/w)% glycerol.

100 µl of competent cells thus prepared were transferred to a 2-mm electro-cuvette, and then 3 µg of pTSa-GX1979 plasmid was added thereto, followed by transformation via electroporation (2.4 kV, 200 Ω, 25 µF). The transformed cells were resuspended in 1 ml of HS medium containing 2% glucose, and then transferred to a 14-ml round-tube, followed by incubation at 30°C and 160 rpm for 2 hours. Then, the cells were spread on a plate containing an HS-agar medium supplemented with 2% glucose, 1(v/v)% ethanol, and 5 µg/ml tetracycline, and cultured at 30°C for 5 days.

### (3) Test of Glucose Consumption and Cellulose Production

The strain cultured in (2) was inoculated into a 250-mL flask containing 25 ml of HS medium supplemented with 5% glucose, 1% ethanol, and 5 µg/ml tetracycline, and cultured at 30°C and 230 rpm for 5 days. As a result, cellulose (hereinafter, also referred to as "cellulose nanofiber (CNF)") was formed on the surface where the medium was in contact with air. CNF thus produced was collected as a pellicle, washed with 0.1 N NaOH and distilled water at 60°C, and then freeze-dried to remove H₂O, followed by weighing.

Glucose and gluconate were analyzed by HPLC equipped with an Aminex HPX-87H column (Bio-Rad, USA). Table 1 shows CNF production and yield, gluconate yield, and glucose consumption of the *K. xylinus* strain introduced with an Fba gene.

**[Table 1]**

| Strain | CNF production (g/L) | CNF yield (g/g) | Gluconate yield (g/g) | Glucose consumption (g/L) |
|---|---|---|---|---|
| pTSa-EX1 | 1.36 | 0.07 | 1.16 | 18.9 |
| pTSa-GX1979 | 1.96 | 0.07 | 0.99 | 27.1 |

In Table 1, pTSa-EX1 represents a pTSa-EX1 vector-containing *K.xylinus* strain used as a control group, and pTSa-GX1979 represents a pTSa-GX1979 vector-containing *K.xylinus* strain used as an experimental group. As shown in Table 1, the *K. xylinus* strain (pTSa-GX1979) transformed with the FBA GX_1979 gene showed a 43.6% increase in CNF production, as compared with the pTSa-EX1 control strain. In Table 1, CNF yield and gluconate yield represents grams of CNF/gram of glucose and grams of gluconate/gram of glucose, respectively. As shown in Table 1, the experimental group and the control group showed similar CNF yields. The experimental group showed a 25% decrease in gluconate yield, as compared with the control group. Further, the experimental group showed a 43.0% increase in glucose consumption, as compared with the control group. The glucose consumption represents glucose g/medium 1 L.

**Example 2: Introduction of phosphoglucomutase gene or UTP-glucose-1-phosphate uridylyltransferase gene**

Glucose-6-phosphate produced via gluconeogenesis is converted to UTP-glucose which is a substrate of cellulose synthase by phosphoglucomutase (hereinafter, also referred to as "PGM") and UTP-glucose-1-phosphate uridylyltransferase (hereinafter, also referred to as "UGP") enzymes. In this Example, *K.xylinus* was introduced with a *K. xylinus* DSM2325 M9 strain-derived PGM gene or UGP gene, and effects of the gene introduction on cellulose productivity were examined.

### (1) Construction of vector

Open reading frames of the PGM GX_1215 gene (SEQ ID NO: 3) and the UGP GX_2556 gene (SEQ ID NO: 5) were obtained by gel extraction after PCR using genomic DNA of the *K.xylinus* DSM2325 M9 strain as a template and a set of primers of either SEQ ID NOS: 14 and 15 or SEQ ID NOS: 16 and 17. The obtained ORF of each gene was cloned into a Sall restriction site of a pTSa-EX11 vector using an In-Fusion GD cloning kit (Takara) to prepare an overexpression vector. Hereinafter, these vectors are referred to as a pTSa-GX1215 vector and a pTSa-GX2556 vector, respectively.

### (2) Transformation

*K. xylinus* DSM2325 M9 strain was spread on a plate containing an HS-agar medium supplemented with 2% glucose, and cultured at 30°C for 3 days. The strain thus cultured was flushed with 2 ml of sterile water, and colonies were pooled. The colonies were transferred to a 50 ml falcon tube, followed by vortexing for 2 minutes. The 2% glucose-containing HS-agar medium included 0.5% peptone, 0.5% yeast extract, 0.27% Na₂HPO₄, 0.15% citric acid, 2% glucose, and 1.5% bacter-agar. Thereafter, 1% cellulase (sigma, Cellulase from Trichoderma reesei ATCC 26921) was added and allowed to react at 30°C and 160 rpm for 2 hours. Then, the colonies were washed with 1 mM HEPES buffer-containing medium and then washed with 15(w/w)% glycerol three times, followed by resuspension in 1 ml of 15(w/w)% glycerol.

100 µl of competent cells thus prepared were transferred to a 2-mm electro-cuvette, and then 3 µg of pTSa-GX1215 or pTSa-GX2556 plasmid was added thereto, followed by transformation via electroporation (2.4 kV, 200 Ω, 25 µF). The transformed cells were resuspended in 1 ml of HS medium containing 2% glucose, and then transferred to a 14-ml round-tube, followed by incubation at 30°C and 160 rpm for 2 hours. Then, the cells were spread on a plate containing an HS-agar medium supplemented with 2% glucose, 1(v/w)% ethanol, and 5 µg/ml tetracycline, and cultured at 30°C for 5 days.

**(3) Test of Glucose Consumption and Cellulose and Gluconate Productions**

The strain cultured in (2) was inoculated into a 250-mL flask containing 25 ml of HS medium supplemented with 5% glucose, 1% ethanol, and 5 µg/ml tetracycline, and cultured at 30°C and 230 rpm for 5 days. As a result, cellulose (hereinafter, also referred to as "cellulose nanofiber (CNF)") was formed on the surface where the medium was in contact with air. CNF thus produced was harvested as the pellicle and washed with 0.1 N NaOH and distilled water at 60°C, then freeze-dried to remove H₂O, and then weighed.

Glucose and gluconate were analyzed by HPLC. Table 2 shows CNF production, gluconate production, and glucose consumption of the *K. xylinus* strains transformed with either the PGM gene or UGP gene.

**[Table 2]**

| Strain | CNF production (g/L) | Gluconate production (g/L) | Glucose consumption (g/L) | CNF yield (g/g) | Gluconate yield(g/g) |
|---|---|---|---|---|---|
| pTSa-EX1 | 1.61 | 25.14 | 26.83 | 0.06 | 0.94 |
| pTSa-GX1215 | 1.82 | 25.25 | 26.39 | 0.07 | 0.96 |
| pTSa-GX2556 | 2.10 | 29.22 | 34.47 | 0.06 | 0.85 |

In Table 2, pTSa-EX1 represents a control strain of a pTSa-EX1 vector-containing *K.xylinus* strain, pTSa-GX1215 represents an experimental PGM-transformed strain of a pTSa-GX1215-containing *K.xylinus* strain, and pTSa-GX2556 represents an experimental UGP-transformed strain of a pTSa-GX2556 vector-containing *K.xylinus* strain. As shown in Table 2, the PGM gene- and UGP gene-introduced strains respectively showed increases of 13.0% and 30.4% in CNF production, as compared with the control group, respectively.

It should be understood that embodiments described herein should be considered in a descriptive sense only and not for purposes of limitation.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A recombinant microorganism of the genus *Gluconacetobacter* having cellulose productivity, the recombinant microorganism comprising a genetic modification that increases fructose-bisphosphate aldolase (FBA) activity.

2. The microorganism of claim 1, wherein the genetic modification is an increased copy number of a gene encoding the fructose-bisphosphate aldolase.

3. The microorganism of claim 2, comprising an exogenous gene encoding the fructose-bisphosphate aldolase.

4. The microorganism of claim 2 or 3, wherein the fructose-bisphosphate aldolase belongs to EC 4.1.2.13.

5. The microorganism of any one of claims 1-4, wherein the fructose-bisphosphate aldolase is a polypeptide having a sequence identity of 95% or more with respect to an amino acid sequence of SEQ ID NO: 1.

6. The microorganism of claim 2 or 3, wherein the gene has a nucleotide sequence of SEQ ID NO: 2.

7. The microorganism of any one of claims 1-6, wherein the microorganism is *Gluconacetobacter xylinus.*

8. The microorganism of any one of claims 1-7, further comprising one or more genetic modifications selected from the group consisting of a genetic modification that increases the activity of phosphoglucomutase (PGM), which catalyzes conversion of glucose-6-phosphate to glucose-1-phosphate; a genetic modification that increases the activity of UTP-glucose-1-phosphate uridylyltransferase (UGP), which catalyzes conversion of glucose-1-phosphate to UDP-glucose; and a genetic modification that increases the activity of cellulose synthase (CS), which catalyzes conversion of UDP-glucose to cellulose.

9. The microorganism of claim 8, wherein the microorganism has an increased copy number of one or more genes selected from the group consisting of a gene encoding phosphoglucomutase, which catalyzes conversion of glucose-6-phosphate to glucose-1-phosphate; a gene encoding UTP-glucose-1-phosphate uridylyltransferase, which catalyzes conversion of glucose-1-phosphate to UDP-glucose; and a gene encoding cellulose synthase, which catalyzes conversion of UDP-glucose to cellulose.

10. The microorganism of claim 8 or 9, wherein the microorganism has an increased copy number of one or more of (i) a gene encoding a phosphoglucomutase having 95% or more sequence identity to SEQ ID NO: 4; and/or (ii) a gene encoding a UTP-glucose-1-phosphate uridylyltransferase having 95% or more sequence identity to SEQ ID NO: 6.

11. The microorganism of any one of claims 8-10, wherein the microorganism has an increased copy number of one or more of (i) a gene having a nucleotide sequence of SEQ ID NO: 3 and/or (ii) a gene having a nucleotide sequence of SEQ ID NO:5.

12. A method of producing cellulose, the method comprising:
culturing the recombinant microorganism of any one of claims 1-11 and
collecting cellulose from the culture.

13. The method of claim 12, wherein the recombinant microorganism comprises an increased copy number of a gene encoding a fructose-bisphosphate aldolase.

14. A method of producing the microorganism of any one of claims 1-11, the method comprising introducing a genetic modification that increases fructose-bisphosphate aldolase (FBA) activity into a microorganism of the genus *Gluconacetobacter.*
